(19) Europäisches Patentamt
European Patent Office
Office européen des brevets

(11) **EP 3 669 783 A1**

(12) **EUROPEAN PATENT APPLICATION**

(43) Date of publication:
**24.06.2020 Bulletin 2020/26**

(21) Application number: **18215305.6**

(22) Date of filing: **21.12.2018**

(51) Int Cl.:
*A61B 6/00* (2006.01)        *G21K 1/04* (2006.01)
*G21K 1/06* (2006.01)        *G01N 23/041* (2018.01)
*G02F 1/165* (2019.01)        *G01V 5/00* (2006.01)

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(71) Applicant: **Koninklijke Philips N.V.
5656 AG Eindhoven (NL)**

(72) Inventors:
• **STEADMAN BOOKER, Roger
5656 AE Eindhoven (NL)**
• **ROESSL, Ewald
5656 AE Eindhoven (NL)**

(74) Representative: **de Haan, Poul Erik et al
Philips International B.V.
Philips Intellectual Property & Standards
High Tech Campus 5
5656 AE Eindhoven (NL)**

(54) **SWITCHABLE PHASE STEPPING**

(57)    Phase stepping for differential phase contrast and/or dark field x-ray imaging using a switchable grating in which particles in a reservoir are aligned into wall-like x-ray absorbing structures by inducing a standing wave in a medium in the reservoir. The standing wave is modified by a second ultrasound generator that modifies the standing wave such that the pressure nodes of the first standing wave shift position.

FIG. 3

# Description

## FIELD OF THE INVENTION

The present invention generally relates to a device and method for phase stepping a grating for differential phase contrast and/or dark field x-ray imaging, a switchable grating and a phase contrast imaging procedure.

## BACKGROUND OF THE INVENTION

Phase contrast imaging, such as dark-field x-ray imaging (DAX) and differential phase contrast imaging (DCPI), provide high sensitivity to phase-gradients and scattering structures in the object and are a promising addition to diagnostic or analytical (x-ray) imaging, for instance for medical diagnoses, material analysis and security scanning.

Phase contrast imaging (this term is used throughout this document to cover both DAX and DCPI) is an imaging technique that only recently found practical use in medical imaging. Phase contrast imaging has already been long known for visual optics, but for x-ray imaging it was restricted to highly brilliant synchrotron x-ray sources that are not suitable for medical imaging due to their size and very limited energy band width and angular divergence. However, a grating-based solution was developed to generate dark field x-ray images using x-ray tubes commonly used in medical imaging. See for instance: Pfeiffer et.al., "Hard-X-ray dark-field imaging using a grating interferometer", Nature Materials, Vol. 7, February 2008, page 134-137].

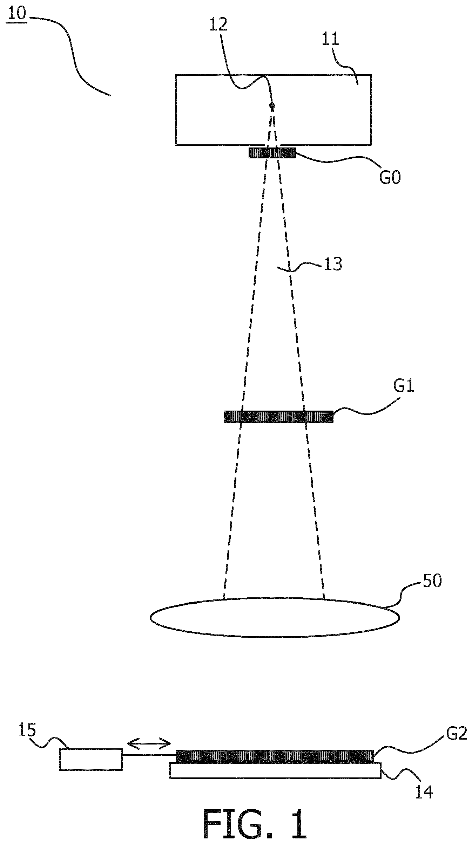

Such grating-based phase contrast imaging may be performed using a phase contrast set-up 10 as is schematically depicted in fig. 1. This set-up is called a Talbot-Lau interferometric arrangement. An X-ray beam 13 is emitted from an x-ray focal spot 12 of an x-ray source 11. A first grating structure G0, commonly named the source grating, is placed close to the focal spot 12. Because of the source grating G0 the radiation beam 13 is in effect divided into an array of line sources splitting up the beam into separate parallel beams, which then pass through a second grating structure G1, commonly named the phase grating, which may be placed in front (e.g. for computed tomography (CT) imaging), or behind (e.g. for standard x-ray imaging) a subject 50 to be imaged. The x-ray beam 13 passes a third grating structure G2, commonly named the analyzer grating, before it is detected by a detector 14, in which imaging information is generated and transmitted to processing equipment (not shown). Both the phase grating G1 and the analyzer grating G2 contribute to image contrast, wherein the phase grating G1 causes periodic spatial phase modulations in the x-ray beam 13. By propagation of the partially coherent x-rays, the phase modulation is transformed into an intensity modulation which has a typical period in the range of 5 - 50 μm (Talbot effect). The analyzer grating G2 then transforms the high-frequency intensity modulations into a low-frequency intensity modulation. When the phase grating G1 or the analyzer grating G2 is moved transversely with respect to the radiation beam 13, the x-ray intensity detected oscillates in each pixel of the detector 14, wherein the magnitude of the oscillations is determined by the subject 50. These local intensity changes may then be used to determine dark field and phase contrast image data, which are obtained simultaneously.

The obtained dark field image data represents scatter information of the x-ray beam 13 through the subject 50. This scatter data is obtained simultaneously with x-ray transmission image data, which provides attenuation measurement data, particularly of a difference between high and low absorption areas, and with phase contrast image data, which provides increased soft-tissue contrast, which makes it particularly suitable for imaging 'soft' materials with many surface area transitions and/or micro-structures (e.g. lungs, fibrous materials and the like).

The obtained differential phase contrast image represents refractive index information of the x-ray beam 43 through the subject 50. This may be advantageously used, on its own or in combination with the simultaneously obtained transmission image, to enhance image contrast by using detailed differing refractive index changes within structures that are otherwise uniform.

The described phase contrast set-up is very suitable for this purpose, but the presently claimed invention would work with any phase contrast set-up suitable for medical, analytical or security imaging based on gratings.

To be able to take multiple acquisitions at different positions over the period of the grating (pitch) in phase contrast imaging a process called phase stepping is often employed. In this, preferably, the phase grating G1 (but this could also be any one of the other two gratings) is "stepped", in other words: the grating is slightly shifted in a direction perpendicular to the x-ray beam 13. Phase stepping is a necessity in most of currently existing differential phase contrast setups making use of Talbot-Lau interferometry. The stepping is typically implemented by an actuator that activates any of the three gratings of a Talbot-Lau interferometer with respect to the two others in synchrony with the readout of the X-ray detector sensing the changes in intensity at various locations within the field-of-view induced by the stepping.

The activation leads to a positional shift of the grating. After the shifting of the grating the X-ray detector is read out. Therefore, the operator acquires a readout prior to the shifting and after the shifting.

An example of a phase stepping device is described in US 2015/0294749 A1. The interferometric dynamic grating is actuated by a microelectromechanical system (MEMS) to change its periodicity. A movable part of the dynamic grating is anchored by springs on two lateral sides of the grating in the direction of movement of the grating. Comb drive means on the sides of the grating allow for modification of the grating in the desired direction. The comb drive means may be piezo-electrically or electrostatically driven.

Known disadvantages of known phase stepping devices include possible delays which are required before the X-ray readout of each phase step can be triggered in view of a possible time it takes the actuator to settle at the new position. Furthermore, positional inaccuracies, back-lash, etc. may occur.

Further, the use of such ultra-precision actuators requires time-consuming installation and extensive calibration steps and is easily disturbed by outside influences, e.g. mechanical disturbances or thermal influences. Also, the actuators and their controls take up valuable space in or near the examination area of the imaging device. And further, if an imaging system is to be used in normal, attenuation-based imaging then the gratings and support devices need to be moved outside of the beam field of view.

Therefore it would be advantageous to obtain a new phase stepping device for phase contrast imaging that overcomes the above-mentioned drawbacks.

SUMMARY OF THE INVENTION

The presently claimed invention provides a solution to the above-mentioned problems and more.

Embodiments according to the present invention are directed towards a phase stepping device for differential phase contrast and/or dark field x-ray imaging comprising a switchable grating. The switchable grating comprises a reservoir containing a substantially x-ray transparent medium comprising particles that substantially attenuate x-ray radiation; a first ultrasound generator, arranged at and acoustically connected to a first side of the reservoir, configured to generate a first soundwave such that a first standing wave is formed within the medium causing the x-ray absorbing particles to organize along pressure nodes of the standing wave; and a second ultrasound generator and acoustically connected to the reservoir configured to generate a second soundwave such that a second standing wave is formed within the medium causing the x-ray absorbing particles to organize along pressure nodes of the standing wave. The switchable grating is configured to shift a position of the pressure nodes between at least two positions by generating the first standing wave by the first ultrasound generator and the second standing wave by the second ultrasound generator in a predetermined sequence. Using both ultrasound generators allows to shift the pressure nodes, and therewith the organized particles, to another position. As such the position of the formed x-ray absorbing structures may be shifted in a predetermined sequence between at least two (preferably three and more preferably four) positions and the switchable grating may therefore be used for phase stepping. An advantage thereof is that no mechanical actuation means need to be implemented and /or the switchable grating does not need to be moved itself, thereby avoiding errors in repositioning.

In the context of the presently claimed invention when the term 'shifting the position of the pressure nodes' is mentioned it is meant that the row or grid of pressure nodes is shifted laterally as a whole.

In an embodiment the predetermined sequence is a sequence in which the first ultrasound generator and the second ultrasound generator are switched on and off alternatingly such that only one is switched on at the time or a sequence wherein the first ultrasound generator is switched on while the second ultrasound generator is alternatingly switched on and off. In the first option the grating position is determined by one ultrasound generator at the time, each producing a (different) standing wave with a different pressure node position. This is possible if the ultrasound generators are positioned differently or are operating at different settings (e.g. frequency or phase). In the second option the second ultrasound generator causes the standing wave caused by the first ultrasound to change such that the pressure node position is changed.

In an embodiment the second ultrasound generator is arranged along a second side of the reservoir opposite to the first side. When the second ultrasound generator is also switched on it generates a second soundwave in the opposite direction of the first soundwave causing the position of the pressure nodes of the resulting soundwave to change with respect to the original node position in the original soundwave. This is a very precise and easily controllable embodiment that results in well-defined and exactly positioned pressure nodes along which the x-ray absorbing particles organize.

In an embodiment the reservoir comprises a first sub-reservoir and a second sub-reservoir stacked on the first sub-reservoir and shifted laterally with respect to the first sub-reservoir. The first ultrasound generator is arranged at and acoustically connected to a first side of the first sub-reservoir and the second ultrasound generator is arranged at and acoustically connected to a first side of the second sub-reservoir. As such each ultrasound generator may form a soundwave in each sub-reservoir. As the sub-reservoirs are shifted with respect to each other the pressure nodes of the standing wave of each sub-reservoir are shifted with respect to each other. As such the x-ray absorbing walls formed by organized particles maybe formed alternatively in the first sub-reservoir and the second sub-reservoir, causing them to shift position each time one ultrasound generator is switched on and the other off, which makes this arrangement very suitable for phase stepping.

In an embodiment the reservoir comprises at least one further sub-reservoir, stacked on and shifted laterally with respect to the previous sub-reservoir, wherein a further ultrasound generator is arranged at and acoustically connected to a first side of the further sub-reservoir. Stacking one or more laterally shifted sub-reservoir allows for phase stepping between multiple (sub)-positions.

In an embodiment a further ultrasound generator is arranged at and acoustically connected to a second side, opposite of the first side, of at least one, but preferably all, of the sub-reservoirs. This allows phase stepping within one-sub-reservoir as well, thereby further extending the amount of possible stepping positions.

In an embodiment the first ultrasound generator and the second ultrasound generator are phase locked, preferably by means of phase locked loop electronic circuits. This ensures formation of a stable standing wave.

In an embodiment the phase stepping device further includes an acoustic diode arranged to prevent a soundwave of an ultrasound generator to reach the opposite side of the reservoir. This prevents distortions in the standing wave that may influence the stability or positioning of the organized particles.

Preferably the acoustic diode comprises a compartment containing a first bubbly liquid and a compartment containing a second bubbly liquid placed in series between the ultrasound generator and the reservoir. This is a preferred implementation of an acoustic diode that provides sufficient dampening of reflections.

The invention further is directed towards a switchable grating as used in the claimed phase stepping device described previously.

The invention is further directed towards a method of phase stepping a grating using the phase stepping device and switchable grating as claimed and described previously.

The invention is further directed towards a phase contrast imaging procedure including the phase stepping method as claimed and described previously.

Still further aspects and embodiments of the present invention will be appreciated by those of ordinary skill in the art upon reading and understanding the following detailed description. Numerous additional advantages and benefits will become apparent to those of ordinary skill in the art upon reading the following detailed description of preferred embodiments.

BRIEF DESCRIPTION OF THE DRAWINGS

The present invention is illustrated by drawings of which:

Fig. 1 shows a schematic representation of a grating interferometer for phase contrast imaging.
Fig. 2 shows a schematic representation of a switchable grating using ultrasound to organize particles in a medium with the ultrasound generator (a) switched off, (b) operating at a first frequency, (c) operating at a second frequency).
Fig. 3 shows a schematic representation of a switchable grating with two opposing ultrasound generators that is used as a phase stepping device as claimed in (a) perspective view, (b) side view.

Fig. 4 shows a schematic representation of an acoustic diode in a (a) negative direction, (b) positive direction.
Fig. 5 shows a schematic representation of a switchable grating that is used as a phase stepping device with two acoustic diodes as claimed.
Fig. 6 shows schematic representations of a switchable grating with stacked sub-reservoirs that is used as a phase stepping device as claimed.
Fig. 7 shows a schematic representation of a switchable grating that is used as a phase stepping device as claimed with two pair of ultrasound generators (a) operating in a first direction, (b) operating in a second direction.

The invention may take form in various components and arrangements of components, and in various process operations and arrangements of process operations. The drawings are only for the purpose of illustrating preferred embodiments and are not to be construed as limiting the invention. To better visualize certain features may be omitted or dimensions may be not be according to scale.

DETAILED DESRIPTION OF EMBODIMENTS

The insight underlying the presently claimed invention is that phase stepping may be performed with a so-called ultrasound switchable grating, as are for instance known from US2007/0183584A1. Herein a temporary grating structure is formed in a medium by means of ultrasound standing waves that allow the alignment of attenuating particles (e.g. Au) along one direction into three dimensional walls or slabs along formed pressure nodes perpendicular to the ultrasound wave direction. The advantage of this is that a grating may be formed on demand in desired dimensions without the need to introduce and position the grating structure into a beam path of an x-ray imager. Also this provides an alternative for currently highly expensive manufacturing costs to produce gratings suitable for phase contrast imaging. Adapting and using such a grating in a novel and inventive way, as will be elaborated on extensively in the following, results in phase stepping enforced by changing the phase shift between opposing traveling ultrasound waves forming a standing wave. The difference in phase determines the location of the pressure nodes. As such this known switchable grating technology is modified electronically to the effect of changing the effective phase, i.e. phase stepping.

The presently claimed invention is described with a focus on the analyzer grating G2 as being the switchable grating 20. However, the switchable grating 20 as claimed may also be used for the source grating G0 or the phase grating G1 or for two or all three of the gratings G0, G1, G2.

The presently claimed invention is described with a focus on the phase contrast arrangement as shown in Fig. 1 as described earlier, but could also be used for alternate arrangements, such as a phase contrast set-up with less phase gratings or where the phase grating G1 is placed behind the object 50.

The detector 14 employed in the phase contrast arrangement according to the presently claimed invention may have a pitch sufficiently small, hence a spatial resolution sufficiently large, for detecting, i.e. adequately resolving the interference pattern generated by the phase grating G1. For that purpose such detection unit may comprise a high resolution x-ray detector 14 known per se having a resolution of 50 micrometers or more, or an x-ray detector 14 of the type as described in US 2014/0177795 A1. Alternatively, the detection unit may comprise an x-ray detector 14 having less high resolution, however, in conjunction with an analyzer grating G2, i.e. an absorption grating arranged in the optical path between the phase grating G1 and said x-ray detector 14.

In case no analyzer grating G2 is used, then also G0 may optionally be removed.

The interferometer employed in the phase contrast arrangement according to the present invention may be implemented by various geometries. The interferometer may comprise (i) (optionally, depending on the x-ray source) a source grating G0 with pitch po, (ii) a phase grating G1 with pitch $p_1$ and installed between the source grating G0 and the detector 14, and (iii) (optionally, depending on the implementation of the detection unit 14) an analyzer grating G2 with pitch $p_2$ and installed between the phase grating G1 and the detector. Introducing s as the distance between the source grating G0 and the analyzer grating G2 (if any), 1 as the distance between the source grating G0 and the phase grating G1 and d as the distance between the phase grating G1 and the analyzer grating G2 (if any), the various geometries are defined on the basis of said quantities. As a first option, the interferometer may be implemented in the so-called "conventional geometry" in which $1 > d$ and $p_0 > p_1 > p_2$. In the conventional geometry, the object to be imaged is typically arranged between the source grating G0 and the analyzer grating G1. As a second option, the interferometer maybe implemented in the so-called "inverse geometry" in which $1 < d$ and $p_0 < p_1 < p_2$. In the inverse geometry, the object to be imaged is typically arranged between the phase grating G1 and the x-ray detection unit 14 (i.e. between the phase grating G1 and, if present, the analyzer grating G2). As a third option, the interferometer may be implemented in the so-called "symmetric geometry" in which $d = 1$ and $p_0 = p1 = p_2$ (presuming a $\pi$-shifting phase grating G1). For more information (incorporated herein by reference) see Tilman Donath et al, "Inverse geometry for grating based x-ray phase contrast imaging", JOURNAL OF APPLIED PHYSICS 106, 054703, 2009."

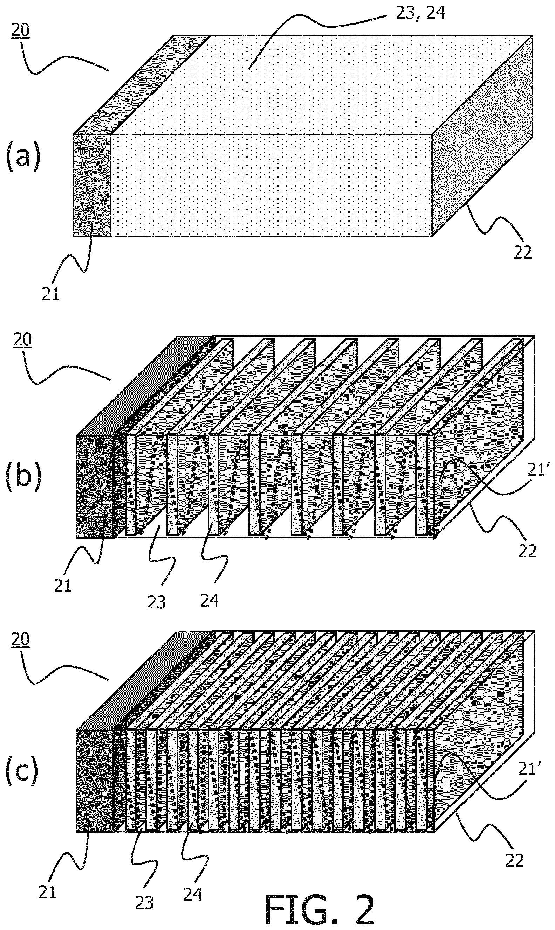

Fig. 2a schematically depicts a known ultrasound switchable grating 20 comprising a reservoir 22, filled with a suspension of (preferably micrometer or nanometer sized) x-ray absorbing particles 24 in a medium 23, such as for instance regular water or another liquid which has negligible x-ray absorption, such as for instance oils, salt water, distilled water, acetone, alcohol and mixtures thereof. The choice of medium is part of the degrees of freedom of the system. An adequate medium may be chosen to achieve an equivalent speed of sound that allows a practical implementation. Gold particles are particularly suitable as x-ray absorbing particles 24, since they have high absorption and no solubility in most liquids, including water. Other materials, such as tungsten, molybdenum, silver or combinations of materials, polymeric materials, for instance blended or filled with metals, may be suitable as well.

The reservoir 22 is at least acoustically, connected to an ultrasound generator 21.

Preferably, the distance between the two sides of the reservoir 22 are better integer multiples of half the wavelength of the induced standing wave ($\lambda$/2). Waves will still form in other cases, but if this condition is met, the standing waves are much stronger because it then operates as a resonance cavity with larger pressure amplitudes, which results in increased focusing of particles 24 in the pressure nodes.

The reservoir 22 has a minimum thickness to allow the particles 24 to arrange to the full height to form grating structures. The height is comparable with the length of regular, non-switchable gratings used in phase contrast imaging, preferably between 100 and 300 micron, more preferably between 150 and 250 micron and most preferably about 200 micron. Preferably the standing soundwave forces the particles 24 to organize in structures substantially extending from the bottom to the top reservoir to the top. However it may be possible that there remains a non-used volume above the grating structures in the reservoir 22. The thickness of the reservoir 22 should increase or decrease if the used particles 24 absorb less or more respectively than a common grating.

The reservoir 22 should be constructed of a material that has negligible x-ray absorption. Glass is a particularly suitable material. Also most plastics would be suitable, provided that the walls are sufficiently thin. The thickness of the reservoir walls should be such that there is a balance between reducing x-ray absorption and structural integrity.

Upon exposing the suspension 23, 24 to a strong ultrasound signal 21' generated by the ultrasound generator 21, standing waves are generated causing the particles 24 to align along pressure nodes of the standing wave, as is schematically shown in figure 2b. By properly selecting an excitation frequency and phase, the particles 24 will from slabs or walls along the volume of the medium separated by the distance between adjacent pressure nodes (i.e. half the ultrasound wavelength). For example, the speed of sound in water is approximately 1500 m/s. If an ultrasound source of 25 MHz is used, the particles will align and form pillars at 30 $\mu$m intervals:

$$\lambda/2 = \frac{c}{2v} = \frac{1500\frac{m}{s}}{2 \cdot 25\,MHz} = 30\mu m.$$

During ultrasound excitation the walls or slabs remain in place, forming an equivalent grating with an absorbing pitch of 30 μm that is suitable for use as an analyzer grating G2. For a sufficiently absorbing cross-section, the ultrasound transducer(s) must be such that a standing wave is generated across the full volume of the suspension exposed to the x-ray beam.

The ultrasound generator 21 comprises an ultrasound transducer that is used to stablish a standing wave 21' across the water (or other) filled container. The ultrasound transducer may be a single transducer or consist of a plurality of smaller transducers each tuned to generate a standing wave.

In an example the particles 24 are gold particles. The medium 23 is water and the cross-section of the reservoir 22 is 0,2 mm. Fig. 2a the transducer is off and all particles 24 are scattered across the medium 23. In this embodiment use is made of reflections at the opposite wall of the reservoir 22 to force a standing wave. As discussed later, it will be beneficial to actively remove the particles away from the field of view when the transducer is switched off. As soon as the transducer is switched on (Fig. 2b), the particles 24 align each pressure node (30 μm) forming gold walls alternating with equally sized water layers.

An interesting additional useful property of ultrasound generated grating structures is the flexibility of their formation and modification by means of proper beam forming techniques. For example, the modification of the speed of sound of the suspension, e.g. by dilution would cause the speed of sound to change and with it the periodicity of the structures formed. The same effect can be achieved by change in the ultrasound frequency. The addition of higher harmonics with the correct phase and amplitude by means of Fourier synthesis might be used to manipulate and improve the profile of the resulting attenuating walls (steeper profile).

As an example, in fig. 2c the ultrasound 21 is operated such that a standing wave 21' such that gold walls with a smaller pitch, e.g. 15 μm, are formed.

In the above described ultrasound-induced switchable grating the frequency of the standing wave and the speed of sound of the medium determine the pitch of the resulting absorbing walls or slabs. It is an insight underlying the presently claimed invention that in a similar way, the phase stepping can be implemented.

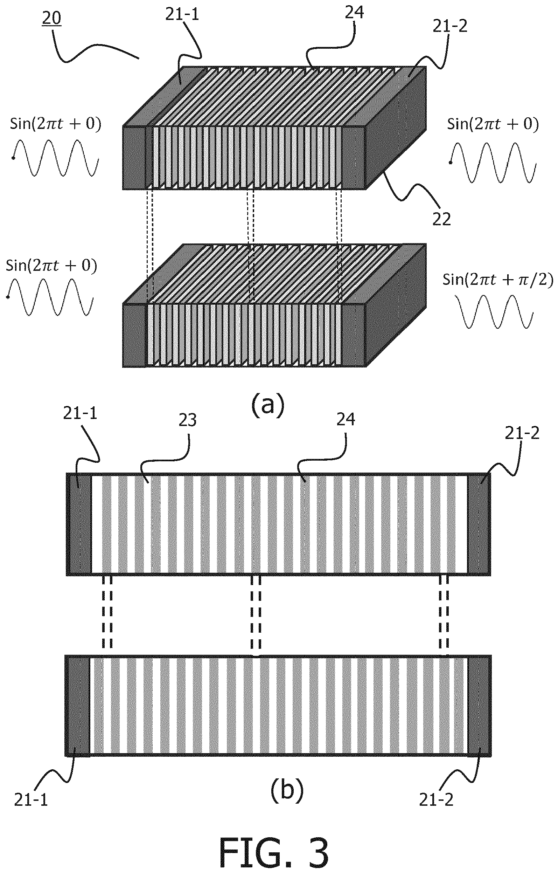

Although phase stepping can be done at any of the three gratings, it makes most sense to consider phase stepping the analyzer grid G2. At a given frequency and medium, the position of the walls across the reservoir 22 depends on the standing wave. Phase stepping may be accomplished by modifying the phase of the resulting ultrasound standing wave. The use of two ultrasound generators 21-1, 21-2 each comprising radio frequency (RF) transducers on opposite sides of the reservoir 22 may be used to modify the phase of the resulting standing wave. That is, when both ultrasound generators 21-1, 21-2 are switched on, opposing ultrasound travelling waves result in a standing wave where a position of the pressure nodes in the medium will depend on the phase difference of both RF transducers. Both RF transducers operate at the same frequency and are phase locked to the desired difference in phase. See fig. 3a with corresponding cross-sections in fig. 3b. In both these figures the top figure depicts the situation in which transducers both ultrasound generators 21-1, 21-2 operate in the same phase and in the same frequency [sin(2πt + 0)], while in the bottom pictures they are operated in the same phase, but a different frequency [sin(2πt + 0) and sin(2πt + π/2)]. As such the location of pressure nodes of the standing wave is shifted, resulting in repositioning of the walls of x-ray absorbent material. The dotted lines between the top and bottom drawings in figs. 3 a and 3b shows that in this example the walls 24 and spaces consisting of the medium 23 switched places upon the operating frequency change of the RF transducer of the second ultrasound generator 21-2.

An added advantage of the present invention is that there is a large degree of freedom in the amount of shift that may be induced. Shifts may be in a positive or negative direction and set to any desired stepping distance if physically possible. It is for instance also possible to increment the phase shift in small increments to a desired stepping distance. It is therefore possible to relocate the walls of absorbing particles on demand at different positons, effectively allowing for phase stepping without mechanical parts or moving the grating itself.

The difference in phase may be realized very precisely by electronics means. For instance, Phase Locked Loop (PLL) circuits force both transducers to sync to the same frequency while allowing to precisely tune the phase difference.

In the embodiment of the switchable grating 20 as shown in figs. 3a and 3b it is assumed that there are no reflections bouncing back from the surface of the RF transducers of the ultrasound generators 21-1, 21-2 that are in contact with the medium 23 in the reservoir 22. This is a necessary condition in most ultrasound applications and is commonly resolved by ensuring an equivalent impedance matching of the transducers to the medium 23 (i.e. the medium is modelled like a transmission line to that effect). Alternatively, the reflections may be exploited to achieve the standing wave.

There are also other known ways of preventing ultrasound reflections, such as use of a so-called acoustic diodes 30 that allow unidirectional wave propagation. Such acoustic diodes 30 prevent the wave to reach the transducer at the other side by blocking its propagation. Such an acoustic diode was disclosed in C. Vanhille and C. Campos-Pozuelo in "Ultrasounds in bubbly liquids: Unidirectional propagation and switch", Physics Procedia 63 (2015), pages 163-166. This particular acoustic diode 30 makes used of sound propagation properties of sound in bubbly liquids.

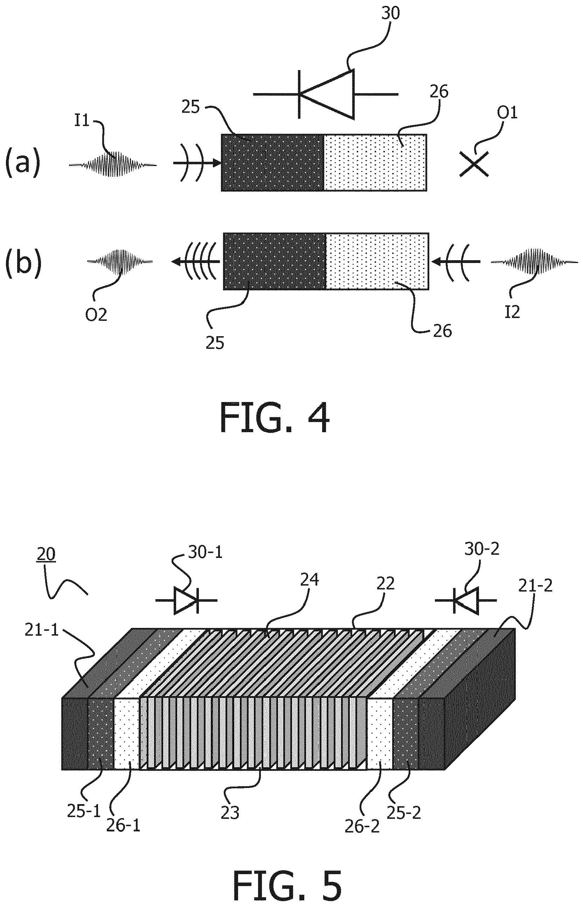

The basic principle is shown in figs. 4a and 4b. Two compartments 25, 26, each with a bubbly liquid (e.g. water) but with different bubble sizes, are placed in series in a direction of the (to be generated) input (ultra-)soundwave I1, I2. The bubbly liquids are tuned to the frequency of the intended soundwave.

In fig. 4a the 'negative' direction is depicted. The input soundwave I0 propagates through the bubbly liquid in the first compartment 25, which disperses the soundwave and has a large band of attenuation around the bubble resonance. This dampens the soundwave and the output signal O1 is therefore negligible.

In fig. 4b the 'positive' direction is depicted. In this the input signal I2 enters the acoustic diode 30 from the opposite side through the bubbly liquid in the second compartment 2, which elevates the higher frequencies (second harmonics) and the (ultra-)sound signal still contains the input frequencies and their second harmonics. As such it propagates into the bubbly liquid in the first compartment 25, which still attenuates the original input frequencies, but does not filter the higher (second harmonic) frequencies. Therefore there is an output signal O2 is that passed through the bubbly liquid in the first compartment 25, albeit at a different frequency than that of the original input signal 12.

Fi. 5 shows a schematic depiction of an implementation of acoustic diodes 30-1, 30-2 as described above in a switchable grating 20 for phase stepping according to the presently claimed invention. In this embodiment a first acoustic diode 30-1 comprises a bubbly liquid in a first compartment 25-1 directly adjacent to the first ultrasound generator 21-1 and a bubbly liquid in a second compartment 26-1 between the first compartment 25-1 and the reservoir 22 and wherein the bubbly liquids have different bubble sizes. The second acoustic diode 30-2 comprises a bubbly liquid in a second compartment 25-2 directly adjacent to the second ultrasound generator 21-2 and a bubbly liquid in a second compartment 26-2 between the second compartment 25-2 and the reservoir 22 and wherein the bubbly liquids have different bubble sizes. As such both acoustic diodes 30-1, 30-2 have a 'positive' direction with respect to an ultrasound wave propagating from the ultrasound generator 21-1, 21-2 it is directly adjacent to and has a 'negative' direction for an ultrasound wave propagating from the ultrasound generator 21-1, 21-2 on an opposite side of the reservoir 22. The ultrasound waves are therefore dampened after they contributed to generating the standing wave in the medium 23 forming the x-ray absorbing walls 24 and no reflections occur that may disturb the position or consistency of the x-ray absorbing walls 24. As the output ultrasound wave O2 now is different form the input ultrasound wave 12, the pressure nodes are now formed based on the second harmonics of the original ultrasound wave and the ultrasound generators 21-1, 21-2 must therefore be set such that this is taken into account to obtain the desired wall structures.

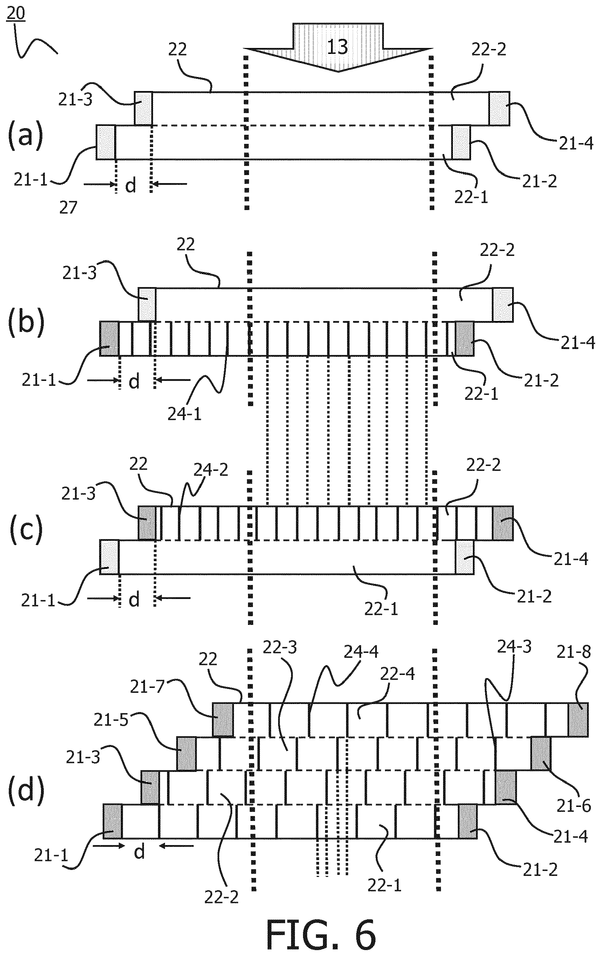

An alternative embodiment of the phase stepping device 20 as claimed is shown in fig. 6. In this embodiment the reservoir 22 is sub-divided into at least two sub-reservoirs 22-1, 22-2, 22-3, 22-4. The sub-reservoirs 22-1, 22-2, 22-3, 22-4 are stacked on top of each other in the direction of an incoming x-ray beam 13 when switched on and are shifted laterally in a direction perpendicular to the incoming x-ray beam 13 when switched on. In this example only two sub-reservoirs 22-1, 22-2 are stacked, but for imaging performance the arrangement preferably consists of at least three stacked sub-reservoirs, more preferably 4 or more. This embodiment is not limited to just two stacked sub-reservoirs 22-1, 22-2 as is shown in figs. 6a, 6b and 6c or to four stacked sub-reservoirs 22-1, 22-2, 22-3, 22-4 as is shown in fig. 6d. Any desired number of stacked sub-reservoirs is, within practical reason, possible.

The sub-reservoirs 22-1, 22-2, 22-3, 22-4 may individually be separated by an x-ray transparent wall to prevent mobility of particles 24 between sub-reservoirs to ensure homogeneous operation and x-ray absorption for each sub-reservoir 22-1, 22-2, 22-3, 22-4.Optionally these separating walls are acoustically dampened to prevent x-ray absorbing structures from being formed in neighboring sub-reservoirs.

Each sub-reservoir is acoustically connected to a separate ultrasound generator 21-1, 21-3, 21-5, 21-7 on a first side of the sub-reservoir. Each ultrasound generator 21-1, 21-3, 21-5, 21-7 may independently induce a standing wave in the sub-reservoir 22-1, 22-2, 22-3, 22-4 it is connected to and, preferably, not substantially in neighboring sub-reservoirs 22-1, 22-2, 22-3, 22-4. As such, in each sub-reservoir a standing wave with a different pressure nodes position may be induced, causing the x-ray absorbing particles 24-1, 24-2, 24-3, 24-4 to organize in walls that have a different position in each sub-reservoir 22-1, 22-2, 22-3, 22-4 in case one or more of the ultrasound generators 21-1, 21-3, 21-5, 21-7 are switched on.

In a preferred embodiment each ultrasound generator 21-1, 21-3, 21-5, 21-7 operates at the same settings and therefore generates the same soundwave and standing wave as the other ultrasound generators. In that case, the lateral shift distance d of the sub-reservoirs also results in the same shift in formed pressure node positions, when switched on.

Alternatively each ultrasound generator 21-1, 21-3, 21-5, 21-7 may operate at different settings to induce different soundwaves and standing waves to allow for more variation in pressure node positions and therefore stepping distances. However, this will entail more complex positioning and calibration.

Fig. 6a shows a situation in which the first and second ultrasound generators 21-1, 21-3 are switched off and no x-ray absorbing walls are formed. If there are means to remove the x-ray absorbing particles from the reservoir 22, then this configuration may be used for regular transmission imaging.

Fig. 6b shows a situation in which the first ultrasound generator 21-1 is switched on and the second ultrasound generator 21-2 is switched off. A standing wave is formed in sub-reservoir 22-1 only and x-ray absorbing particles organize themselves into x-ray absorbing walls 24-1 at the position of the pressure nodes of the standing wave.

Fig. 6c shows the opposite situation in which the second ultrasound generator 21-2 is switched on and the first ultrasound generator 21-1 is switched off. A standing wave is formed in sub-reservoir 22-2 only and x-ray absorbing particles organize themselves into x-ray absorbing walls 24-2 at the position of the pressure nodes of the standing wave.

In the example shown in figs. 6b and 6c the ultrasound generators 21-1, 21-2 operate with the same settings, resulting in a standing wave and pressure node position that corresponds to each other. The lateral shift distance d between the sub-reservoirs is half the distance between pressure nodes of the induced standing wave when in operation. As a result phase stepping of the switchable grating is possible between two equidistant positions of absorbing walls 24-1, 24-2. For instance the lateral shift distance d may preferably be a sixteenth wavelength $\lambda$/16, an eight wavelength $\lambda$/8, a fourth wavelength $\lambda$/4 a third wavelength $\lambda$/3 of the standing wave or multitudes thereof to obtain pressure node distances that can be easily set or adapted using the ultrasound generators 21-1, 21-2. Other later shift distances are of course also possible should the situation require this.

Fig. 6d shows an example of an embodiment in which four sub-reservoirs 22-1, 22-2, 22-3, 22-4 are stacked. Each reservoir is laterally shifted with respect to each other with the same lateral shift distance d in this embodiment. Each sub-reservoir 22-1, 22-2, 22-3, 22-4 is equipped and acoustically connected with a separate ultrasound generator 21-1, 21-3, 21-5, 21-7, each set to operate at the same settings to generate a standing wave and pressure node position that correspond to each other. In this example the lateral shift distance d results in four equidistantly spaced phase stepping positions. Of course any stepping positions may be arranged, such as multitudes of $\lambda$/8 or non-uniform stepping position distances. The ultrasound generators 21-1, 21-3, 21-5, 21-7 maybe operated separately or in combination, e.g. all on at the same time to form a grating with the smallest possible distance (as seen in top view) between absorbing walls 24-1, 24-2, 24-3, 24-4, or two at the time, e.g. a first pair of alternating ultrasound generators 21-1, 21-5 while a second pair of alternating ultrasound generators 21-3, 21-7 is switched off and vice versa. Alternate options or sequences, such as pairs of neighboring ultrasound generators, in which ultrasound generators are switched on and off would be clear to the skilled person attempting to create a specific phase stepping sequence.

As is exemplary shown in fig. 6, each sub-reservoir 22-1, 22-2, 22-3, 22-4 may be equipped with a second ultrasound generator 21-2, 21-4, 21-6, 21-8 on a second side of the sub-reservoir opposite the first side. As such each sub-reservoir would also be able to operate as a switchable grating and phase stepping device on its own as described previously in relation to the embodiment illustrated in fig. 3, providing even further options and possibilities for phase stepping distances and sequences.

Preferably each sub-reservoir 22-1, 22-2, 22-3, 22-4 is equipped with means for flushing particles from the field of view (indicated by the thick dotted lines in fig. 6) of the x-ray beam 13 to prevent unwanted x-ray attenuation by x-ray particles in any of the sub-reservoirs for which the ultrasound generator is not switched on. This may be achieved in many ways, for instance, by flushing the medium to a further reservoir, by sweeping the particles 24 towards a side of the sub-reservoir (e.g. using a membrane-like structure) or by generating a propagating ultrasound wave from the ultrasound generator 21 that sweep the particles to a side of the reservoir outside the field-of-view of the x-ray beam.

Each sub-reservoir 22-1 22-2, 22-3, 22-4 and connected ultrasound generator 21-1, 21-2, 21-3, 21-4, 21-5, 21-6, 21-7, 21-8 may also have an acoustic diode as described previously associated with them.

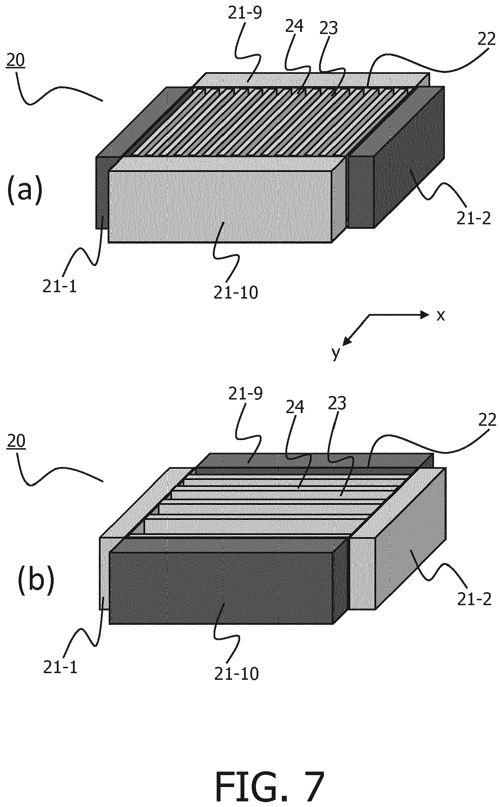

In a further embodiment of the phase stepping device 20 an additional pair of ultrasound generators 21-9, 21-10 are placed on adjacent walls of the reservoir 22, as is schematically depicted in fig. 7. As with the first pair of ultrasound generators 21-1, 21-2, the transducers of the additional ultrasound generators 21-9, 21-10 need to be phase locked with each other. In fig. 7a only the first two ultrasound generators 21-1, 21-2 are switched on, causing x-ray absorbent walls 24 to be formed in the medium 23 in the same manner and direction x as in the previously described embodiments. In fig. 7b only the other two ultrasound generators 21-9, 31-4 are switched on, causing x-ray absorbent walls 24 to be formed in the medium 23 in the same manner, but in a direction y orthogonal to the situation of fig. 7a and the previously described embodiments.

This allows to selectively extend phase stepping to the possibility of rotating the direction of the phase resolution by 90° (which requires rotating the other two gratings in the interferometer with the same amount, either mechanically or by the same electronics method). In this embodiment, only a pair of (opposing) transducers are active at any given time. In a further embodiment, all four transducers are switched on to implement inclinations of the structures by small angles. Depending on the used frequency and phase of each pair of ultrasound generators 21-1/21-2, 21-9/21-10 x-ray absorbent pillars are formed at pressure nodes caused by the two overlapping, orthogonal standing waves.

Acoustic diodes 30 may also be added to the second pair of ultrasound generators 21-9, 21-10 to avoid or reduce reflections of propagating ultrasound waves that may influence the formation and location of the x-ray absorbent grating structures 24.

In this example the orthogonally placed ultrasound generators (21-9, 21-10) were shown for the embodiment of two oppositely placed ultrasound generators (21-9, 21-10), but it would also be possible to adapt this to an embodiment based on stacked ultrasound generators, as depicted and described in relation to fig. 6.

Use of ultrasound generators 21-1, 21-2 as described in all embodiments (or even two orthogonal pairs 21-1/21-2, 21-9/21-10) as described previously results in fast, precise and fully electronic phase stepping.

Imagers equipped with a phase contrast grating arrangement and phase stepping device 20 may be used for regular transmission imaging as well. As mentioned previously, a transmission image is obtained simultaneously with the differential phase contrast and dark field signal, but the signal is incomplete due to attenuation of the gratings G0, G1, G2. For instance, when an analyzer grating G2 is present, then about 50% of the radiation reaching the analyzer grating G2 is attenuated just before it reaches the detector 14. This is particularly problematic when the imager is used for medical transmission x-ray imaging, since 50% of the dose that already passed the object is not used for the actual imaging, thereby unnecessarily exposing the object to too much harmful ionizing radiation.

To avoid this, the grating(s) G0, G1, G2 must be removed from the system, but this will cause extensive repositioning and calibration when the grating arrangement is used again for a later imaging procedure. With the known switchable grating, in the off-state the particles 24 are in suspension or they may have precipitated to the bottom of the reservoir. Since the x-ray absorbing particles still remain in the path of the x-ray beam 13, even in the off-state, they have a non-negligible x-ray absorption for radiation that already passed the object and to avoid this the known switchable grating must still be removed from the path of the radiation beam 13.

To obtain a true transmission image without attenuation by the x-ray absorbing particles 24 in the reservoir 22, they should be removed from the path of the x-ray beam when the transducer is switched off. It is therefore preferable to ensure that they are displaced out of the field-of-view of the x-ray beam 13. This may be achieved in many ways, for instance, by flushing the medium to a further reservoir, by sweeping the particles 24 towards a side of the reservoir (e.g. using a membrane-like structure) or by generating a propagating ultrasound wave from the ultrasound generator 21 that sweep the particles to a side of the reservoir outside the field-of-view of the x-ray beam.

In a method for phase contrast imaging using a device and method according to the presently claimed invention, first, the first ultrasound generator 21-1 is switched on (101) and a soundwave propagates into a reservoir filled with a medium 23 and x-ray absorbing particles, causing a standing wave and organization of the particles into x-ray absorbing wall-like structures at pressure nodes at a first node pattern in the medium, thereby forming a grating structure. Said grating structure is part of a phase contrast interferometer in a phase contrast x-ray imaging device.

Next an imaging procedure is started by introducing an object 50 (in case the object 50 was not yet present) in an examination region of the x-ray beam of the imaging device, switching on the x-ray beam and detecting (102) phase contrast imaging information of the object.

After a predetermined time a second ultrasound generator 21-2 opposite the first ultrasound generator 21-1 as described previously, modifies (103) the standing wave, causing the wall structures to shift to a next node pattern in which the nodes are shifted laterally compared to the previous node position. Further phase contrast information of the object is then detected (104).

After a predetermined time the ultrasound generator is switched off or removed (105) such that the standing wave transitions back to its form and the pressure nodes shift back to their initial positions. After which again phase contrast imaging information is obtained (102) of the object.

This sequence is repeated until the whole object 50, or at least the section of interest of the object 50, is imaged.

In an alternative embodiment the method for phase contrast imaging using a device and method according to the presently claimed invention, in a first step, the first ultrasound generator 21-1 is switched on (101) and a soundwave propagates into a first sub-reservoir 22-1 filled with a medium 23 and x-ray absorbing particles, causing a standing wave and organization of the particles into x-ray absorbing wall-like structures 24-1 at pressure nodes in the medium in the first sub-reservoir, thereby forming a grating structure. Said grating structure is part of a phase contrast interferometer in a phase contrast x-ray imaging device.

Next an imaging procedure is started by introducing an object 50 (in case the object 50 was not yet present) in an examination region of the x-ray beam of the imaging device, switching on the x-ray beam and detecting (102) phase contrast imaging information of the object.

After a predetermined time the first ultrasound generator 21-1 is switched off, thereby removing the x-ray absorbing wall structures 24-1 and simultaneously the second ultrasound generator 21-2, in this embodiment stacked above and shifted laterally with respect to first ultrasound generator 21-1 as described previously, is switched on (103), causing a standing wave in a second sub-reservoir 22-2, which is stacked above and shifted laterally compared to the first sub-reservoir 22-1, causing the wall structures 24-1 to form in the second sub-reservoir 22-2. These wall structures 24-2 are shifted laterally compared to the originally formed wall structures 24-1 in the first sub-reservoir 22-1, as seen from above in the direction of the incoming x-ray beam 13. Further phase contrast information of the object is then detected (104).

After a predetermined time the second ultrasound generator 21-2 is switched off and the first ultrasound generator (21-3) is switched (101) on again to form x-ray absorbing walls 24-1 in the original position, after which again phase contrast imaging information is obtained (102) of the object.

This sequence is repeated until the whole object 50, or at least the section of interest of the object 50, is imaged.

Preferably the x-ray absorbing particles 24-1, 24-2 are flushed from the sub-reservoir 22-1, 22-2 when the connected ultrasound generator 21-1, 21-2 is not switched on.

The latter embodiment of the method may easily be expanded to accommodate a sequence of switching on and off ultrasound generators 21-1, 21-2, 21-3, 21-4 associated with more than two stacked and laterally shifted sub-reservoirs 22-1, 22-2, 22-3, 22-4.

While the invention has been illustrated and described in detail in the drawings and foregoing description, such illustration and description are to be considered illustrative or exemplary and not restrictive; the invention is not limited to the disclosed embodiments.

The presently claimed invention is suitable for any kind of x-ray phase contrast imaging, such as 2D x-ray imaging, x-ray tomosynthesis or computed tomography.

The presently claimed invention allows for useful application in a clinical environment such as a hospital. More specifically, the present invention is very suitable for application in imaging modalities such as mammography, diagnostic radiology, interventional radiology and computed tomography (CT) for the medical examination of patients.

In addition, the presently claimed invention allows for useful application in a medical or non-medical (such as an industrial) environment. More specifically, the present invention is very suitable for application in medical scanning, non-destructive testing (e.g. analysis as to composition, structure and/or qualities of biological as well non-biological samples) as well as security scanning (e.g. scanning of luggage on airports).

The terms 'object' or 'subject' should each in light of the present invention be understood as an inanimate object, e.g. a material for structural or other testing or objects for security checks, or a human or animal subject for, for instance, a medical diagnosis imaging scan.

The terms 'first', 'second', 'further', etc. indicate options and are not limited to a particular sequence or order unless specified. The term 'second' may occur without the presence of a 'first'.

The term substantially means at least >50%, preferably >75%, more preferably >85%, even more preferably >90% and most preferably >95%.

Other variations to the disclosed embodiments can be understood and effected by those skilled in the art in practicing the claimed invention, from a study of the drawings, the disclosure, and the appended claims. In the claims, the word "comprising" does not exclude other elements or steps, and the indefinite article "a" or "an" does not exclude a plurality. A single unit may fulfil the functions of several items recited in the claims. The mere fact that certain measures are recited in mutually different dependent claims does not indicate that a combination of these measured cannot be used to advantage.

## Claims

1.  Phase stepping device for differential phase contrast and/or dark field x-ray imaging comprising:

    - a switchable grating (20) comprising:
    - a reservoir (22) containing a substantially x-ray transparent medium (23) comprising particles (24) that substantially attenuate x-ray radiation;
    - a first ultrasound generator (21-1), arranged at and acoustically connected to a first side of the reservoir, configured to generate a first soundwave such that a first standing wave is formed within the medium causing the x-ray absorbing particles to organize along pressure nodes of the standing wave; and
    - a second ultrasound generator (21-2, 21-9) arranged at and acoustically connected to the reservoir 22 configured to generate a second soundwave such that a second standing wave is formed within the medium causing the x-ray absorbing particles to organize along pressure nodes of the standing wave,

    wherein the switchable grating is configured to shift a position of the pressure nodes between at least two positions by generating the first standing wave by the first ultrasound generator and the second standing wave by the second ultrasound generator in a predetermined sequence.

2.  Phase stepping device according to claim 1, wherein the predetermined sequence is a sequence in which the first ultrasound generator (21-1) and the second ultrasound generator (21-2) are switched on and off alternatingly such that only one is switched on at the time or a sequence wherein the first ultrasound generator is switched on while the second ultrasound generator is alternatingly switched on and off.

3.  Phase stepping device according to claim 1 or 2, wherein the second ultrasound generator (21-2) is arranged at and acoustically connected to a second side of the reservoir, (22) opposite to the first side.

4.  Phase stepping device according to any of the previous claims, wherein the reservoir (22) comprises a first sub-reservoir (22-1) and a second sub-reservoir (22-2) stacked on the first sub-reservoir and shifted laterally with respect to the first sub-reservoir, wherein the first ultrasound generator (21-1) is arranged at and acoustically connected to a first side of the first sub-reservoir (22-1) and the second ultrasound generator (22-3) is arranged at and acoustically connected to a first side of the second sub-reservoir (21-1).

5. Phase stepping device according to the previous claim, wherein the reservoir (22) comprises at least one further reservoir (22-3, 22-4), stacked on and shifted laterally with respect to the previous sub-reservoir (22-2, 22-3), wherein a further ultrasound generator (21-15, 21-7) is arranged at and acoustically connected to a first side of the further sub-reservoir (21-1).

6. Phase stepping device according to claim 4 or 5, wherein a further ultrasound generator (21-2, 21-10, 21-6, 21-8)is arranged at and acoustically connected to a second side, opposite of the first side, of at least one, but preferably all, of the sub-reservoirs (22-1, 22-2, 22-3, 22-4).

7. Phase stepping device according to any of the previous claims, wherein the first ultrasound generator (21-1) and the second ultrasound generator (21-2) are phase locked, preferably by means of phase locked loop electronic circuits.

8. Phase stepping device according to any of the previous claims, further including an acoustic diode (30) arranged to prevent a soundwave of an ultrasound generator (21-1, 21-2) to reach the opposite side of the reservoir (22), preferably the acoustic diode (30) comprises a compartment containing a first bubbly liquid (25) and a compartment containing a second bubbly liquid (26) placed in series between the ultrasound generator (21-1) and the reservoir (22).

9. Switchable grating (20) comprising:

- a reservoir (22) containing a substantially x-ray transparent medium (23) comprising particles (24) that substantially attenuate x-ray radiation, wherein the reservoir (22) comprises a first sub-reservoir (22-1) and a second sub-reservoir (22-2) stacked on the first sub-reservoir and shifted laterally with respect to the first sub-reservoir;
- a first ultrasound generator (21-1), arranged at and acoustically connected to a first side of a first sub-reservoir, configured to generate a first soundwave such that a first standing wave is formed within the medium causing the x-ray absorbing particles to organize along pressure nodes of the standing wave; and

- a second ultrasound generator (21-2, 21-9) arranged at and acoustically connected to a first side of a second sub-reservoir (22-2) configured to generate a second soundwave such that a second standing wave is formed within the medium causing the x-ray absorbing particles to organize along pressure nodes of the standing wave.

10. Switchable grating (20) according to claim 10, wherein the reservoir (22) comprises at least one further sub-reservoir (22-3, 22-4), stacked on and shifted laterally with respect to the previous sub-reservoir (22-2, 22-3), wherein a further ultrasound generator (21-15, 21-7) is arranged at and acoustically connected to a first side of the further sub-reservoir (21-1).

11. Switchable grating (20) according to claim 9 or 10, wherein a further ultrasound generator (21-2, 21-10, 21-6, 21-8)is arranged at and acoustically connected to a second side, opposite or perpendicular to of the first side, of at least one, but preferably all, of the sub-reservoirs (22-1, 22-2, 22-3, 22-4).

12. Method for phase stepping a grating for during a differential phase contrast and/or dark field x-ray imaging with an imaging device comprising a grating interferometer including at least one switchable grating (20) comprising a reservoir (22) containing a substantially x-ray transparent medium (23) comprising particles (24) that substantially attenuate x-ray radiation;
a first ultrasound generator (21-1) arranged at and acoustically connected to a first side of the reservoir and a second ultrasound generator (21-2) arranged at and acoustically connected to the reservoir;
wherein the method comprises the steps of:

- generate (101) a soundwave with the first ultrasound generator (21-1) with a first frequency and first phase such that a standing wave is formed within the medium causing the x-ray absorbing particles to organize along pressure nodes of the standing wave; and
- generate (102) a second soundwave with the second ultrasound generator (21-2) such that a second standing wave is formed within the medium causing the x-ray absorbing particles to organize along pressure nodes of the standing wave causing the pressure nodes of the standing wave to shift position.

13. Method according to the previous claim, further comprising:

modify (105) the standing wave causing the pressure nodes of the standing wave to shift to their original position by switching the first ultrasound generator (21-1) and second ultrasound generator (21-2) on and off alternatingly such that only one is switched on at the time or a sequence wherein the first ultrasound generator is switched on while the second ultrasound generator is alternatingly switched on and off; repeat steps (b) and (c) during the differential phase contrast and/or dark field x-ray imaging procedure.

14. Method according to claim 12, further comprising:

   (c) switching of the first ultrasound generator (21-1) prior to switching on the second ultrasound generator and wherein the switchable grating (20) is a switchable grating according to any of the claims 9 to 11.

15. Method according to any of the claims 12 or 13, wherein at least one further standing wave is generated using at least one further ultrasound generator (21-9, 21-10, 21-5, 21-6, 21-7, 21-8) is arranged at a first ultrasound generator (21-1) arranged at and acoustically connected to a first side of the reservoir a side of the reservoir (22).

16. Phase contrast imaging procedure comprising the steps of:

   - (a) obtaining phase contrast information (102) of an object (50) with a phase contrast imaging device comprising a grating interferometer comprising at least one grating arranged to switch between at least two positions and;
   - (b) change the position (103) from a first position to a second position of the at least one grating using the phase stepping method of any of the claims 12 to 14;
   - (c) obtaining phase contrast information (104);
   - (d) change the position (105) from the first position to the second position of the at least one grating using the phase stepping method of claim 13;
   - (e) repeat steps (a) to (d) until phase contrast information of at least a section of interest of the object (50) has been obtained.

# FIG. 1

FIG. 2

$Sin(2\pi t + 0)$

$Sin(2\pi t + 0)$

$Sin(2\pi t + 0)$

$Sin(2\pi t + \pi/2)$

(a)

(b)

FIG. 3

FIG. 4

FIG. 5

# FIG. 6

FIG. 7

Europäisches
Patentamt

European
Patent Office

Office européen
des brevets

# EUROPEAN SEARCH REPORT

Application Number

EP 18 21 5305

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | US 2007/183584 A1 (BAUMANN JOACHIM [DE] ET AL) 9 August 2007 (2007-08-09) * the whole document * ----- | 1-16 | INV. A61B6/00 G21K1/04 G21K1/06 G01N23/041 G02F1/165 |
| A | US 2007/189449 A1 (BAUMANN JOACHIM [DE] ET AL) 16 August 2007 (2007-08-16) * the whole document * ----- | 1-16 | ADD. G01V5/00 |
| A | DE 955 476 C (KOCH & STERZEL AG) 3 January 1957 (1957-01-03) * the whole document * ----- | 1-16 | |

TECHNICAL FIELDS
SEARCHED (IPC)

A61B
G01V
G21K
G01N
G02F

The present search report has been drawn up for all claims

| Place of search | Date of completion of the search | Examiner |
|---|---|---|
| Munich | 24 June 2019 | Anscombe, Marcel |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 18 21 5305

This annex lists the patent family members relating to the patent documents cited in the above-mentioned European search report.
The members are as contained in the European Patent Office EDP file on
The European Patent Office is in no way liable for these particulars which are merely given for the purpose of information.

24-06-2019

| Patent document cited in search report | Publication date | Patent family member(s) | Publication date |
|---|---|---|---|
| US 2007183584 A1 | 09-08-2007 | DE 102006037282 A1<br>JP 2007203060 A<br>US 2007183584 A1 | 09-08-2007<br>16-08-2007<br>09-08-2007 |
| US 2007189449 A1 | 16-08-2007 | DE 102006037257 A1<br>JP 2007206076 A<br>US 2007189449 A1 | 02-08-2007<br>16-08-2007<br>16-08-2007 |
| DE 955476 C | 03-01-1957 | NONE | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 20150294749 A1
- US 20070183584 A1
- US 20140177795 A1

### Non-patent literature cited in the description

- **PFEIFFER.** Hard-X-ray dark-field imaging using a grating interferometer. *Nature Materials,* February 2008, vol. 7, 134-137
- **TILMAN DONATH et al.** Inverse geometry for grating based x-ray phase contrast imaging. *JOURNAL OF APPLIED PHYSICS,* 2009, vol. 106, 054703
- **C. VANHILLE ; C. CAMPOS-POZUELO.** Ultrasounds in bubbly liquids: Unidirectional propagation and switch. *Physics Procedia,* 2015, vol. 63, 163-166